Europäisches Patentamt

European Patent Office

Office européen des brevets

(11) Publication number: 0 556 496 A2

# EUROPEAN PATENT APPLICATION

(21) Application number: 92303185.0

(22) Date of filing: 09.04.92

(51) Int. Cl.5: **C12P 21/08**, C12N 15/06, G01N 33/574

(30) Priority: **21.02.92 US 839169**

(43) Date of publication of application:
**25.08.93 Bulletin 93/34**

(84) Designated Contracting States:
**DE FR GB**

(71) Applicant: **DEVELOPMENT CENTER FOR BIOTECHNOLOGY**
**81 Chang Hsing Street**
**Taipei(TW)**

(72) Inventor: **Ma, Jeng**
**3F No.7, Alley 4, Lane 112 Sec.3**
**Hsin Long Road Taipei(TW)**
Inventor: **Loa, Chien-Chang**
**3rd Floor, No.54 Tien-Mu W Road**
**Shih-Lin District, Taipei(TW)**
Inventor: **Chang, Tong-Hsuan**
**4F No.10, 85-Xiang Chang Hsing Street**
**Taipei(TW)**

(74) Representative: **Arthur, Bryan Edward**
**Withers & Rogers 4 Dyer's Buildings Holborn**
**London EC1N 2JT (GB)**

(54) **Antibodies against tumor-associated mucin glycoprotein.**

(57) Disclosed is a monoclonal antibody specific to tumor-associated mucin glycoprotein, and a method as well as a kit for detecting the tumor-associated mucin glycoprotein. The monoclonal antibody is secreted and produced by hybridoma 7D9-233.

## BACKGROUND OF THE INVENTION

The present invention relates to a monoclonal antibody specific to tumor-associated mucin glycoprotein and a hybridoma capable of producing the same, as well as a kit and a method for detecting the tumor-associated mucin glycoprotein.

Mucin glycoproteins are macromolecules having a molecular weight larger than 1,000,000, and comprise essentially the protein backbone of polypeptides, with covalently bound oligosaccharide side chains. The structure therefore, looks like a bottle brush. The main amino acids on the polypeptides are threonine, serine, and proline. The oligosaccharide side chains consist of monosaccharides, which are essentially hexose, fucose, hexosamine, sialic acid, and sulphate ester, and the average number of monosaccharides is usually less than 10. The carbohydrate side chains comprise up to 80% of the molecular weight of the mucin glycoproteins and therefore,the characteristics can be easily determined by the histochemical staining method. However, as the protein backbone are covered by the saccharide side chains, only a weak staining reaction is developed when said histochemical staining method is applied.

Mucin glycoproteins are secreted by the epithelial cells of some organs such as human gastrointestinal tract, lung, uterine cervicex and have their main function is protecting and lubricating the epithelial cells thereunder, inhibiting bacteria, rendering waterproof capability to the mucosa, and maintaining the water equilibrium of the epithelial cells. The mucin glycoproteins secreted by different organs have their own various chemical properties and antigenicity. During the process of malignant transformation, the quality and quantity of the mucin glycoproteins will both change. For example, the histochemical reaction of the mucin glycoprotein secreted by the stomach will change from neutral to acidic, and mucin glycoproteins secreted by the colon will change from strong acidic to weak acidic or to neutral. Also, their antigenicity will change remarkably (Bara J. et al., Antigens of gastric and intestinal mucous cells in human colonic tumors , Brit. J. Cancer 41: 209-221, 1980).

The cancer of gastrointestinal tract of human beings has the highest incidence (25%). Among which, the stomach and colon are the organs that will get cancer most easily (80%). Therefore, among all cancer patients, 20% have cancer of the stomach and colorectum.

The conventional immunological reagent for detecting the cancer of stomach and colorectum are anti-CEA. However, this reagent is not specific to the cancer of stomach and colorectum only. Another reagent, like anti-CA 19-9, although it can also be used in detecting the cancer of stomach and colorectum, it is usually used to detect gall bladder cancer and pancreatic cancer, and therefore is also not specific to the cancer of stomach and colorectum.

## SUMMARY OF THE INVENTION

It is therefore the major object of the present invention to provide a reagent for detecting tumor-associated mucin glycoprotein, especially the mucin glycoprotein associated with the cancer of stomach and colorectum, with high specificity and enhanced sensitivity.

It is another object of the present invention to provide a method of detecting tumor-associated mucin glycoprotein, especially the mucin glycoprotein associated with the cancer of stomach and colorectum. A method that simplifies the procedure and requires less time to perform.

The above objects of the present invention are attained by producing a monoclonal antibody specific to a mucin glycoprotein associated with the majority of colorectum, and therby discovering a new epitope associated with colorectal cancers. The invention can provide a clinical diagnosis with high specificity and enhanced sensitivity through simple detecting procedures.

The compound and composition of the present invention comprises the hybridoma per se, and monoclonal antibody 7D9-233 secreted by which (including polypeptides of variable regions of heavy chains and light chains subtype with disulfide bonds, and their fragments, such as Fab, F(ab)'2, and Fc). The present invention also comprises the method of detecting the mucin glycoproteins using the above compound and composition, and the kit for performing this method.

## DETAILED DESCRIPTION OF THE INVENTION

The compound and the composition of the invention are produced by the conventional cell fusion technique (Chang, T.H., Tsing,S.Y. and Tzeng,S., monoclonal antibodies against Trichomonas Vaginalis, Hybridoma, vol. 5(1), pp.43-51, 1986), which comprises hybridizing both a murine-origin myeloma cell and an antibody-producing B lymphocyte by means of a compound like polyethylene glycol (PEG), culturing the hybridized cells in HAT, and then analyzing the specificity of the antibody by the immunohistochemical staining method and the enzyme-linked immuno-sorbent assay (ELISA). Thereafter, the mucin glycoprotein-specific hybridoma clones thus selected (i.e. 7D9-233) are administered through an intraperitoneal injection into mice to produce ascites. The antibodies are recovered from the ascites by a protein purification technique.

An immunohistochemical staining method according to the present invention involves adding monoclonal antibody 7D9-233 against tumor-associated mucin glycoproteins onto a dewaxed pathological tissue section to allow the first antibody to bind with the specific mucin glycoprotein in said tissue section to form an antigen-antibody complex. After that, a second antibody (anti-mouse antibody) attached to a signal (i.e. an enzyme, a fluorescent reagent and the like) is added and allowed to link with the above complex. If the signal attached to the second antibody is an enzyme, a substrate is used to develop color. Finally, haematoxylin is used to counterstain the cell nucleus to determine whether a specific tumor-associated mucin glycoprotein is present or not and the quantity thereof.

A sandwich immuno-assay of the present invention involves coating the first antibody onto the surface of a solid support and adding a sample of mucin glycoprotein to allow the first antibody to bind with the mucin glycoprotein to form an antibody-antigen complex. Thereafter, the same antibody attached to a signal (i.e. an enzyme, a fluorescent reagent, a radioactive isotope and the like) is added and allowed to link with said antibody-antigen complex. The antibody added may also be allowed to bind to one or more signal-generating portion(s) along with a high affinity complex, such as a streptoavidin-biotin complex. The presence and the amount of mucin glycoprotein can be determined by the signal generated from the sample.

In addition to the hybridoma clones (7D9-233) and the antibody or the various fragments they produce, the present invention also includes a method of detecting the presence of mucin glycoprotein in a sample, and a kit containing reagents formulated for use of the antibody. The reagents can be used to diagnose fresh or fixed tissues, blood, body fluid or other samples taken from a patient's body. The kit used in immunohistochemical staining method comprises (1) 1% BSA blocking solution, (2) 3% hydrogen peroxide solution, (3) a first antibody which is specific to an antigenic epitope of a tumor-associated mucin glycoprotein, (4) a second antibody-signal complex capable of binding to said first antibody, (5) a substrate, and (6) haematoxylin solution. The kit used in ELISA comprises (1) antigen standard concentration reagent, (2) a solid support, (3) a first antibody, (4) a signal-generating device conjugated with the first antibody, (5) a substrate, and (6) a stop reagent. The solid support comprises a microtiter plate, beads, and papers composed of materials like polyethylene chloride, polystyrene, or nitrocellulose suitable for immobilizing appropriate proteins. The signal-generating device includes a fluorescent substance, a radioactive isotope, or an enzyme such as peroxidase, alkaline phosphatase, beta-galactosidase and the like. All of the above-mentioned techniques and conditions of reactions are conventional (Antibody: A Laboratory Manual, Ed. Harlow & David Lane, 1988).

## BRIEF DESCRIPTION OF THE DRAWINGS

This invention is more specifically described by the following illustrative examples with accompanying drawings, wherein:

Fig. 1 is a microphotograph of a formalin-fixed, paraffin-embedded colon cancer tissue sections stained by immunohistochemical staining method, showing that 7D9-233 have reacted with colon cancer ( positive, brownish red color), and did not react with adjacent normal tissues, wherein, blue color indicates the cell nucleus stained by haematoxylin;

Fig. 2 is an ELISA standard curve obtained from the antigen standard concentration reagent with 7D9-233;

Fig. 3 is a Sepharose 4B gel filtration profile of colon cancer associated mucin glycoprotein;

Fig. 4 is a microphotograph of a formalin-fixed, paraffin-embedded stomach cancer tissue section stained by immunohistochemical staining method using monoclonal antibody 7D9-233, showing that signet ring cells in the stomach cancer have a positive reaction (brownish red) and poorly differentiated cancer cells (left, up corner and left side) have a negative reaction, wherein blue color indicates the cell nucleus counterstained by haematoxylin;

Fig. 5 is a microphotograph of a consecutive section of the same tissue in Fig. 4, stained with Anti-CEA, showing that poorly differentiated cancer cells have a positive reaction, and signet ring cells have a negative reaction; and

Fig. 6 is a microphotograph of a consecutive section of the same tissue in Fig. 4, stained with Anti-CA 19-9, showing a few poorly differentiated cancer cells and signet ring cells have a positive reaction.

**Example 1: Preparation and screening of hybridoma 7D9-233**

The mucin glycoprotein extracted from the tissue of the stomach cancer was purified by density gradient ultracentrifugation. The purified mucin glycoprotein 80 µg and the complete Freund's Adjuvant (CFA) in a ratio of 1:1 was emulsified, and then the emulsion was administered to BALB/C mice through an intraperitoneal injection, once every four weeks for a total of three times. The mice were boosted once three days before cell fusion. Three days later, spleen cells were fused with murine-origin myeloma cells of the FO cell line (ATCC CRL 1646) in a ratio of 1:3 using 50% polyethylene glycol-400 (PEG-400). The cells were then diluted to $1\times10^5$ FO cells by suspension in RPMI (HAT, 20% FCS) and inoculated in the wells of a 96-well microtiter plate (0.1 ml per well). After 10 days, the supernatant of the cell culture was tested by the immunohistochemical staining method and an ELISA to select the hybridoma binding with colon cancer-associated mucin glycoprotein, which was cloned by a limiting dilution method. 7D9-233 was the hybridoma selected by these two methods, which secreting monoclonal antibody tested to be IgM, k light chain. This hybridoma can be stored in a culture broth containing 10% DMSO at -70ºC, and can be cultured by using standard mammalian cell culture techniques, such as culturing in 10% fetal calf serum containing RPMI 1640 supplemented with 1 mM glutamate and 50 mM 2-mercaptoethanol.

**Example 2: Immunohistochemical staining method for detecting tumor-associated mucin glycoprotein**

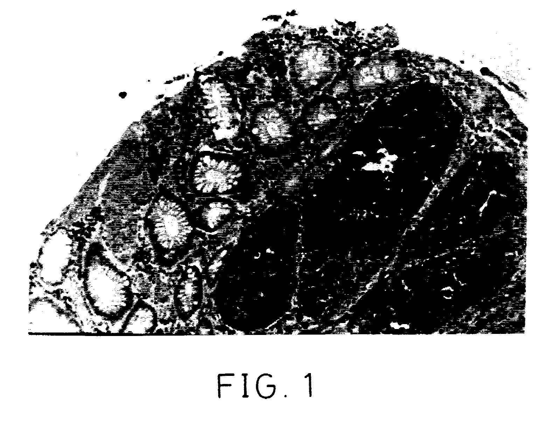

Formalin-fixed, paraffin-embedded pathological tissue sections of stomach cancer, colon cancer, normal stomach and normal colon were dewaxed, and then non-specific bindings were blocked with 1% BSA. After the endogenous peroxidase possibly present in the tissues was blocked by 3% $H_2O_2$, antibody against mucin glycoprotein of colon cancer (7D9-233) were added, washed with buffer, and a second anti-mouse antibody conjugated with horseradish peroxidase was added. After washing, sections developed color with substrate AEC solution, and the nucleus was counterstained with haematoxylin. The microphotograph observed is shown in Fig. 1. Fig. 1 shows that antibody 7D9-233 has reacted with the mucin glycoprotein secreted by the colon cancer cells (positive, brownish) while it did not react with the adjacent morphologically normal tissues. The reactivity of 7D9-233 with colon cancer is 80% (40/50), and the reactivity with epithelial tissues of normal colon is 0% (0/10). Positive reactions are also present for the stomach cancer and normal stomach. These results are shown in Table 1.

Table 1

| The reactivity of 7D9-233 with Human Gastrointestinal Tract | | |
|---|---|---|
| colon cancer | 40/50 | (80%) |
| adjacent Mucosa of colon cancer | 11/38 | (29%) |
| adenoma of the colon | 8/19 | (42%) |
| normal colon | 0/10 | (0%) |
| stomach cancer | 12/18 | (67%) |
| adjacent Mucosa of stomach cancer | 12/17 | (70%) |
| normal stomach | 3/4 | (75%) |
| normal small intestine | 0/4 | (0%) |

For the fetal tissues, 7D9-233 reacted with stomach, small intestine, large intestine, pancereas, lung, gall bladder, and collecting tube in kidney. These results are shown in Table 2.

Table 2

The presence of 7D9-233 reacted epitopes in the fetal tissues

---

1. Positive tissues: stomach, small intestine, large intestine, pancreas, lung, oesophagus, gall bladder, kidney (collecting tube)

2. Negative tissues: heart, liver, skin, muscle, spleen, thymus.

---

Accordingly, we may say that the antigen binding with 7D9-233 is an oncofetal antigen having high specificity to colon cancer.

**Example 3: Sandwich ELISA assay for detecting tumor-associated mucin glycoprotein**

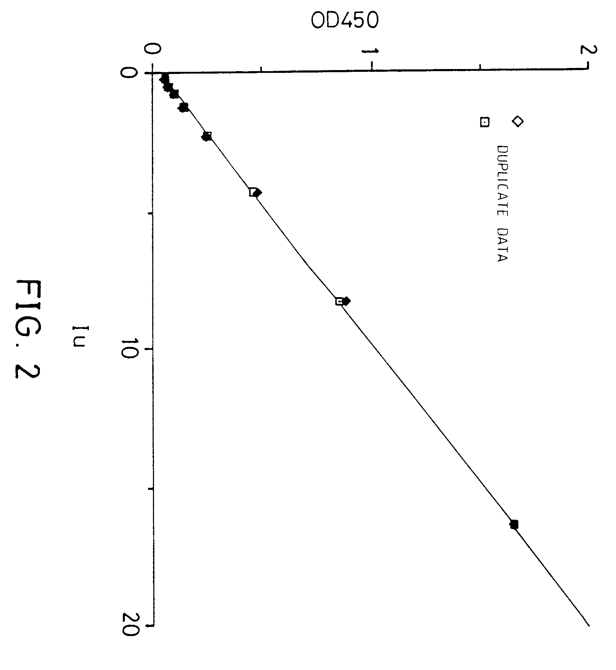

Antibody 7D9-233 was attached to the bottom of each well of a 96-well microtiter plate, and 1% BSA was added to block the non-specific binding. After that, samples may contain colon cancer associated mucin glycoprotein (for example, patient's serum samples) were added, washed, and monoclonal antibody 7D9-233, conjugated with horseradish peroxidase, were also added. After washing, substrate solution TMB was added for color development, and 2N HCl was added to terminate the reaction. Whether colon cancer-associated mucin glycoprotein is present and the amount present were determined by measuring $OD_{450}$ and comparing with the standard curve obtained from the antigen standard concentration reagent. The result is shown in Fig. 2.

**Example 4: Characteristic analysis of colon cancer-associated mucin glycoprotein reacted with 7D9-233**

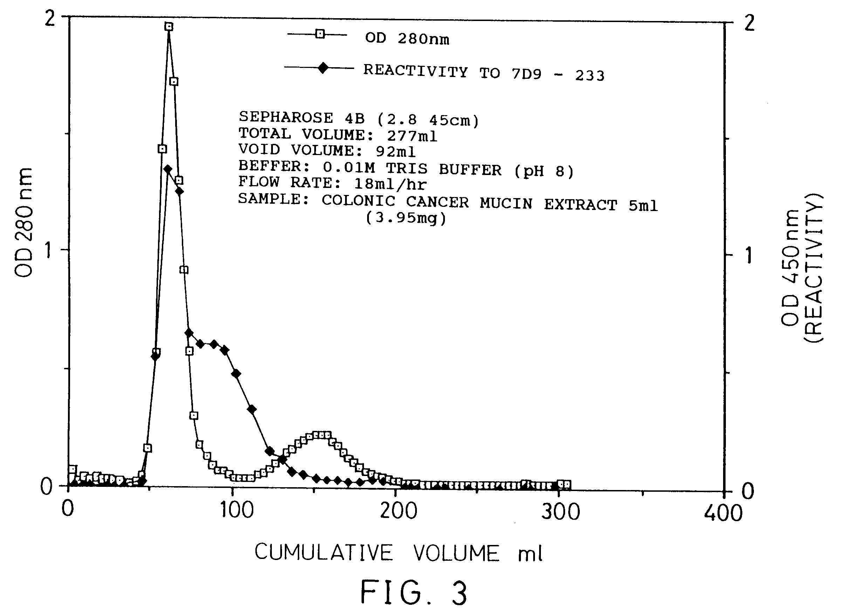

The molecular weight of the colon cancer-associated mucin glycoprotein was determined by Sepharose 4B gel filtration Chromatography to be larger than $1 \times 10^6$ (see Fig. 3). The epitopes reacted with 7D9-233 were found to be repeated epitopes by a sandwich ELISA assay. The nature of epitopes in colon cancer tissues after physical, chemical and enzymatic treatments has shown that the chemical structure of the epitopes reacted with 7D9-233 is a carbohydrate because they resist heat treatment in boiling water up to 15 minutes, resist pronase digestion, survive in the formalin-fixed, paraffin-embedded process, and will be destroyed when exposed to periodate ($NaIO_4$) treatment. These results are shown in Table 3.

## Table 3

Physical, Chemical & Enzymatic Treatments of the Epitopes reacted with 7D9-233

---

1. Heat treatment in boiling water: resistant up to 15 minutes.

2. Periodate oxidation of terminal sugar residues: 50 mM $NaIO_4$ in 0.1 M sodium acetate buffer (pH 5.4) for overnight: destroy the epitopes.

3. Pronase digestion: 6 units/ml in PBS at $37^{O}C$ for 5 hours: resistant to the treatment.

4. Formalin-fixed, paraffin-embedded process: survived

---

### Example 5: Comparison of anti-CEA (BM), anti-CA 19-9 (Histo) and 7D9-233

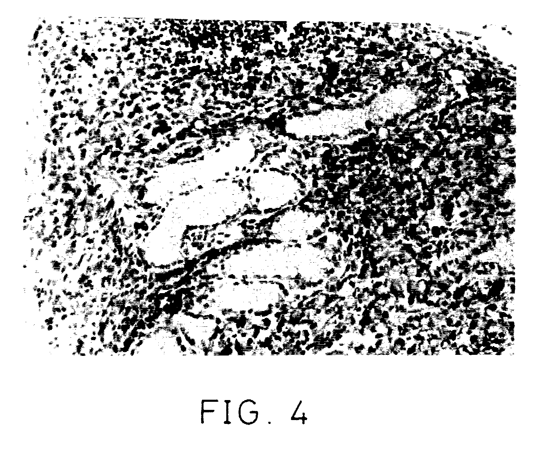

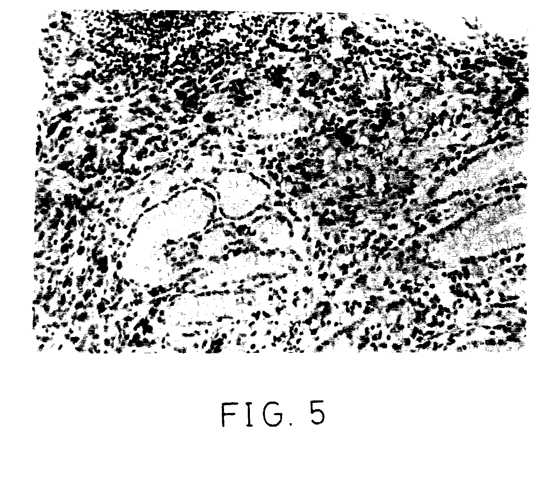

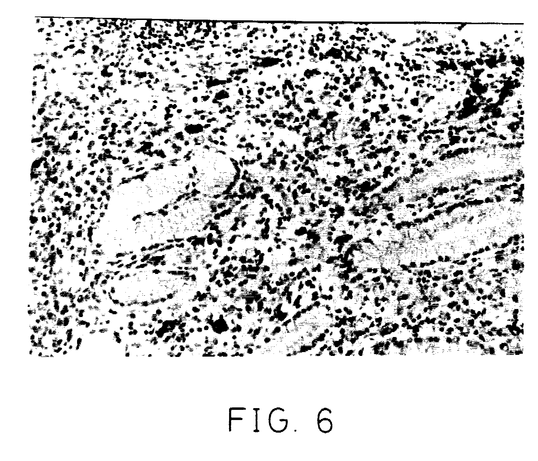

Comparison of anti-CEA (Boehringer Mannheim Co.), anti-CA-19-9 (Histo Co.) and 7D9-233 was carried out by Immunohistochemical staining method, and the results are shown in the microphotograph in Figs. 4, 5, and 6. From the differences between the sites and ratios of positive reactions of the colon cancer tissues and the normal tissues, it is shown that the epitopes identified by 7D9-233 are different from those of anti-CEA and anti-CA 19-9. The results are shown in Table 4.

Table 4

| Reactivity of 7D9-233, anti-CEA and anti-CA19-9 with colon cancer | | | |
|---|---|---|---|
| | 7D9-233 | anti-CEA(BM) | anti-CA19-9(Histo) |
| colon cancer<br>adjacent mucosa | 40/50 (80%)<br>11/38 (29%) | 33/38 (87%)<br>2/26 (8%) | 27/37 (73%)<br>8/25 (32%) |

## Claims

1. A monoclonal antibody specific to tumor-associated mucin glycoprotein.

2. The monoclonal antibody as claimed in claim 1, which is produced and secreted by hybridoma 7D9-233.

3. The monoclonal antibody as claimed in claim 1, which is specific to mucin glycoprotein associated with cancer cells of human gastrointestinal tracts.

4. The monoclonal antibody as claimed in claim 3, which is specific to colon cancer associated mucin glycoprotein.

5. A method for preparing the monoclonal antibody as claimed in claim 1, comprising culturing a hybridoma capable of secreting a monoclonal antibody specific to tumor-associated mucin glycoprotein.

6. The method as claimed in claim 5, wherein said hybridoma is a hybridoma capable of secreting a monoclonal antibody specific to colon cancer associated glycoprotein.

7. The method as claimed in claim 6, wherein said hybridoma is 7D9-233.

8. A polypeptide comprising a variable region of the heavy chains and the light chains of a monoclonal antibody specific to a tumor-associated mucin glycopeptide.

9. The polypeptide as claimed in claim 8, wherein said monoclonal antibody is produced and secreted by hybridoma 7D9-233.

10. A combined composition specific to a tumor-associated mucin glycoprotein, comprising a variable region of a heavy chain and a variable region of a light chain of a monoclonal antibody specific to said mucin glycoprotein.

11. The combined composition as claimed in claim 10, wherein said monoclonal antibody is produced and secreted by hybridoma 7D9-233.

FIG. 1

FIG. 2

FIG. 3

FIG. 4

FIG. 5

FIG. 6